# EUROPEAN PATENT SPECIFICATION

(11) **EP 0 887 052 B1**
(45) Date of publication and mention of the grant of the patent: **17.08.2005**
(21) Application number: 98250211.4
(22) Date of filing: 16.06.1998
(51) Int. Cl.: A61F 2/34

(54) **Screw Socket for an Artificial Hip Joint**
Schraubpfanne für ein künstliches Hüftgelenk
Cupule à vis pour une articulation de hanche artificielle

(30) Priority: 25.06.1997 DE 19727846
(43) Date of publication of application: 30.12.1998
(73) Proprietor: Johnson & Johnson Medical Limited, Ascot, Berkshire SL5 9EY (GB)
(72) Inventor: Witzel, Ulrich Dr., 42279 Wuppertal (DE)
(74) Representative: UEXKÜLL & STOLBERG

(56) References cited:
- EP-A- 0 358 345
- DE-A- 2 950 536
- FR-A- 2 395 011
- US-A- 4 894 064

## Description

The invention concerns a screw socket for an artificial hip joint, according to the preamble of claim 1.

A basic type of a screw socket is known, e.g. from the European Patent Specification EP 0 065 482 B1, which describes a socket with a threaded ring into which a socket inlay of plastics material can be inserted. The thread base of the threaded ring is hemispherical, i.e., it lies on a spherical zone and, in the embodiment example depicted, carries a buttress thread. The thread segments of the self-cutting thread are interrupted by sunk thread grooves through which bone material removed during screwing-in is passed outwards. The thread segments have plane front faces perpendicular to the direction of rotation.

There are extensive studies concerning the screwing-in behaviour of screw sockets, see e.g. "Mechanische Integration von Schraubpfannen" ("Mechanical Integration of Screw Sockets"), Dr. Ulrich Witzel, Georg Thieme Verlag, 1996. When the known screw sockets are screwed into the complementary recess prepared in the acetabulum, the self-cutting thread penetrates the bone material, the screwing-in torque increasing up to a maximum value at which the screw socket has attained the end position, whereby the maximum torque for typical screw sockets and typical bone material can be in the range of 15 to 30 Nm. In the case of altered acetabulum bone material, particularly in the case of the bone material being greatly increased in strength due to sclerosis or calcification, substantially higher torques can be required to screw in the screw socket. In extreme cases, it has happened that it was necessary to apply such high torques that the patient had to be held in position during the screwing-in operation so that he did not turn on the operating table with the screw socket.

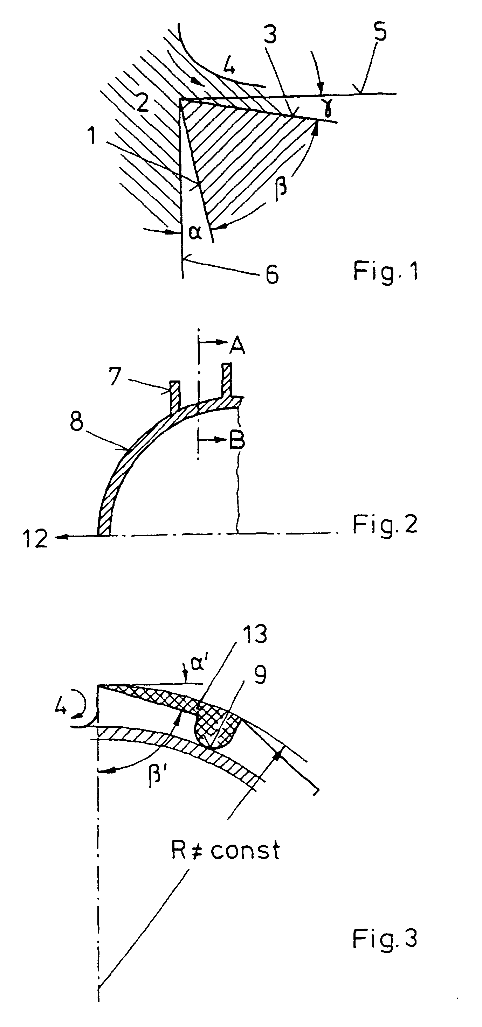

In machine construction, it is known in principle that tool cutting edges can have a ground surface. A cutting tool is depicted schematically in cross section in Figure 1, for the purpose of representing the geometric parameters. In cutting the workpiece 2, the clearance face 1 of the tool forms a clearance angle α with the workpiece surface 6. Located between the cutting face 3 of the tool, over which the cutting 4 is discharged, and the face 5, which is perpendicular to the workpiece surface 6, is the cutting rake γ. The clearance face 1 and the cutting face 3 enclose the wedge angle β.

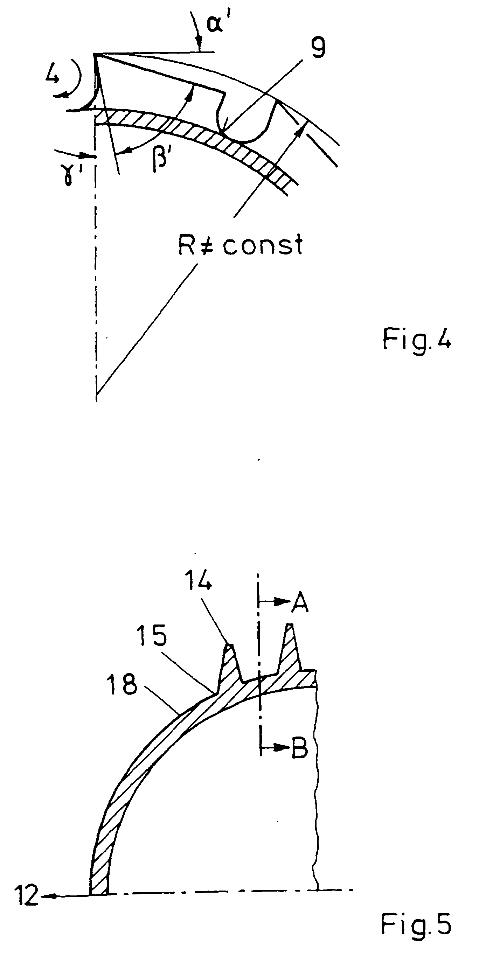

Also known in the existing art are screw sockets with a flat thread, i.e., with thread teeth which are essentially rectangular in the profile cross-section, as depicted in Figure 2, wherein, for the sake of simplicity, only two thread teeth 7 are depicted on the screw socket body 8. Figure 3 shows a section along the plane A-B from Figure 2. In the case of such a flat thread, the cutting 4 being depicted schematically by an arrow in Figure 3, the material-removing cut is effected by an increasing radius R (external radius of the thread contour) due to the pitch of the thread on the screw socket body 8 which can be e.g. conical or, as depicted in Figure 2, hemispherical. The external radius R of the following thread segment, which is separated from the preceding segment by a sunk thread groove 9, is therefore shifted to a somewhat larger radius and consequently again provides for a material-removing cut into the bone material. In the case of screw sockets with a flat thread, embodiments are known in which, in the profile longitudinal section of the thread segments as depicted in Figure 3, the flat thread has a clearance angle α' which is achieved through a rectilinear bevelling of the thread segment. This bevelling produces a loss surface 13, i.e., the surface which is lost to the thread for anchoring in the bone material. There are also known flat threads which, in the profile longitudinal section, are so formed in their front region that they have a cutting rake γ', as depicted in Figure 4.

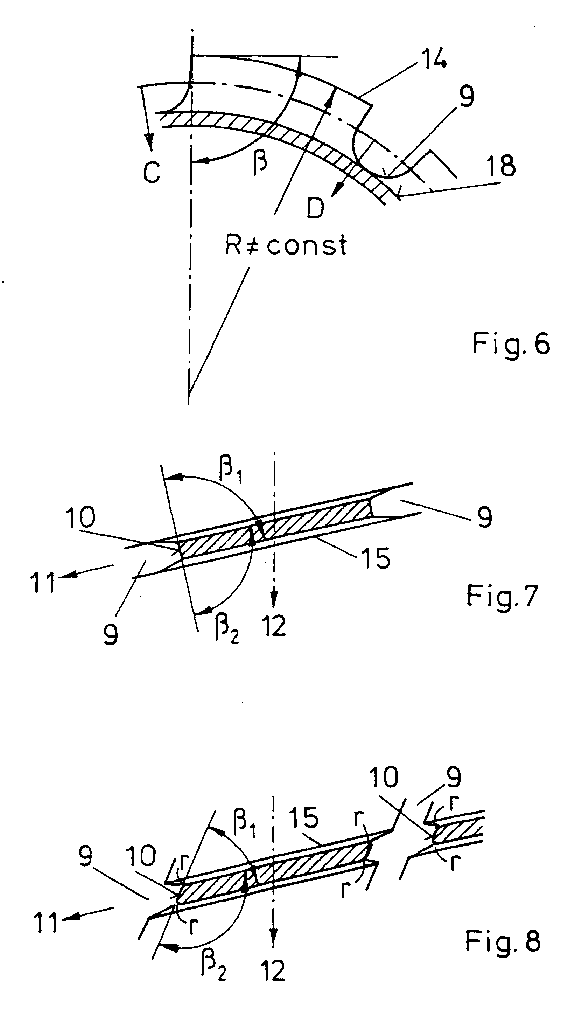

The known V-shaped threads (as also the known buttress, trapezoidal or round threads) on screw sockets do not have any particular cutting edge conformation. Figure 5 depicts, by way of example, a cut-off partial view of a screw socket with a V-shaped thread, wherein, for the sake of simplicity, only two thread teeth are shown in the profile cross-section on the screw socket body. Figure 6 depicts a section along the plane A-B from Figure 5. In the longitudinal view of the thread segments from Figure 6, the V-shaped thread has neither clearance angle α' nor a cutting rake γ'.

Considered below is a further section through the thread segments, namely, along the surface C-D from Figure 6, i.e., essentially a section at constant height above the thread base 18. Such a section is shown in Figure 7, wherein the arrow 12 denotes the rotational axis of the screw socket. On screwing in, the thread segment front face 10 moves in the direction of motion 11. In the case of the thread from Figure 7, there are neither clearance angles α₁, α₂ nor cutting rakes γ₁, γ₂ in this section, i.e., the wedge angles β₁, β₂ are each 90° (the indices 1 and 2 denote the cutting edges on both sides of the front face 10).

In the case of the thread segments fashioned thus, the thread segment front faces primarily displace the bone material upon screwing in. At most, they cut if bone material is forced back into the thread grooves 9. This also applies to thread segments which are separated by thread grooves 9 which run obliquely relative to the direction of screwing in, such as those depicted in Figure 8 which is otherwise the same as Figure 7. Upon screwing in, such threads likewise primarily effect a displacement of the bone material, without a genuine cutting action. In addition, the edges of the thread segments are rounded in the production of the screw sockets, sand-blasting normally being effected for the purpose of surface treatment, as indicated by the radius of curvature r in Figure 8.

Overall, therefore, in the case of screw sockets with a V-shaped thread, as also those with a buttress, trapezoidal or round thread, problems always occur on screwing in if the bone material is very hard due to sclerotic alterations and displacement by the thread is virtually no longer possible. In practice, the problems indicated above occur, in that extremely high torques have to be applied in order to screw the screw socket into its end position.

EP-A-0 358 345 discloses a screw socket according to the preamble of claim 1. The screw socket body has a self-cutting thread, wherein each thread segment in its front region has a cutting face with a clearance face such that, in the section in a plane which is located in the front region with parallel displacement relative to a plane tangential to the thread base, said cutting edge has a clearance angle. In particular such thread shape is described as having each thread segment formed so that its width progressively decreases from its leading end to its trailing end which is said to reduce the tendency of the bone to bind on the trailing portion of the thread segment. Thus the thread segment is tapering from its leading to its trailing end and therefore has clearance angles on the side facing the pole and on the opposite side facing the equator of the socket body.

It is therefore the object of the present invention to create a screw socket with a V-shaped, buttress, trapezoidal or round thread which can be screw into hard bone material with a lesser amount of torque than the screw sockets known hitherto.

This object is achieved by the characterizing features of Claim 1 in association with the pre-characterizing clause of the said claim. Advantageous embodiments of the invention are detailed in the sub-claims.

The clearance angle α₁ and the cutting rake γ₁ are preferably greater than 0.01 (radian measure).

It is particularly preferable to fashion the thread segment cutting edges as spatial cutting edges which not only have a clearance angle α in the first-mentioned section parallel to the thread base, but also, in addition, have a clearance face with a clearance angle α' in the profile longitudinal section (i.e., through a plane located, tangentially relative to the spatial spiral line of the thread course, in the front region of the thread segment). The claimed spatial cutting edge design is completed if the thread segment in the last-mentioned profile longitudinal section also has a cutting rake γ'.

According to a further aspect of the invention, there is created a screw socket for an artificial hip joint with a self-cutting thread on a thread base of a screw socket body having a pole which screw socket is characterized in that each thread segment is formed, in the front region in the direction of rotation, with a cutting edge with a clearance face so that, in the section in a plane which is located in the front region with parallel displacement relative to a plane tangential to the thread base, the said cutting edge has a cutting rake γ and, in this section, is widened in the direction to the pole only in the front region so that, due to the widening, the front cutting edge projects over the envelope of the leading thread segment end in the direction of rotation.

The invention is described below with reference to embodiment examples described in the drawings, wherein:
Figure 1: shows a schematic sectional representation of a tool, for the purpose of explaining the geometric tool parameters;
Figure 2: shows a partial view of a screw socket from the existing art, in the section through a plane containing the rotational axis 12 of the screw socket;
Figure 3: shows a sectional view of the plane A-B from Figure 2;
Figure 4: shows a view as in Figure 3 for another screw socket from the existing art;
Figure 5: shows a partial view of a screw socket, in the section through a plane containing the rotational axis 12, a screw socket from the existing art with a V-shaped thread being depicted;
Figure 6: shows a sectional view along the plane A-B from Figure 5;
Figure 7: shows a section through the thread, along the surface C-D from Figure 6;
Figure 8: shows a representation as in Figure 7 for another known thread;
Figures 9 to 11: show representations of thread segments according to the invention in sectional views corresponding to the view from Figures 3 and 4;
Figure 12: shows a section through the thread, corresponding to the view in Figures 7 and 8, for a screw socket; and
Figures 13 and 14: show views, corresponding to Figure 12, of alternative embodiments according to the invention.

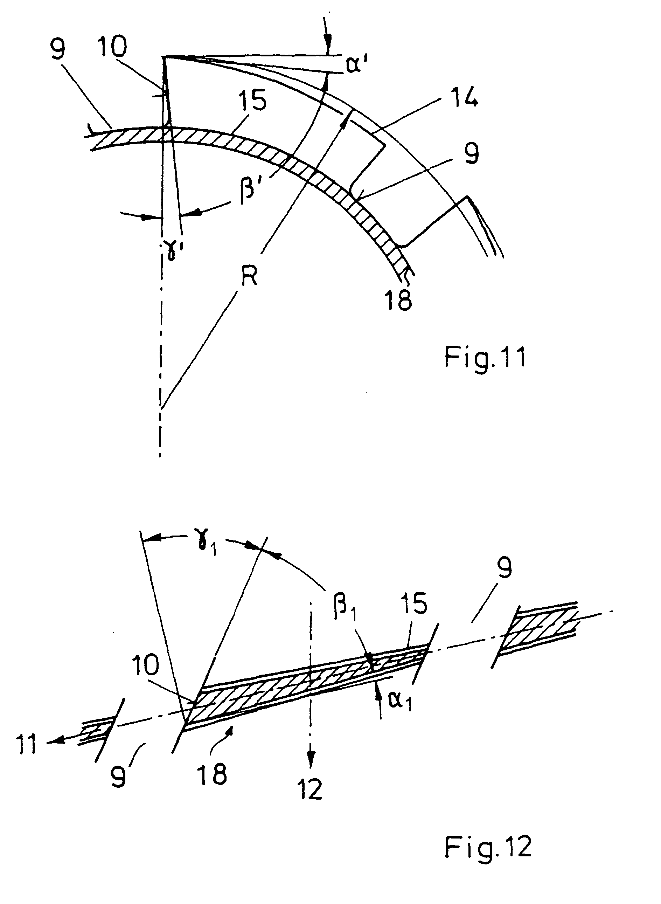

Figure 12 depicts a section through a screw socket, corresponding to the section through the surface C-D from Figure 6, i.e., the thread segments have been cut open at a certain height above the thread base 18. In the case of a hemispherical screw socket, the section through the surface C-D corresponds to a section along a spherical surface. The claims, by contrast, are based on a section through a plane which lies in the front region of the thread segment, with parallel displacement relative to a plane tangential to the thread base. In a first approximation, the last-mentioned sectional representation corresponds locally, in the front region, in the direction of rotation, of the cutting edge of the thread segment, to the section depicted in Figure 12.

In the section depicted in Figure 12, the thread segment tapers away from its front face 10 towards the next thread groove 9. The cut surface of the thread segment is represented by hatched lines and the reference 15 denotes the root lines of the thread segment on the thread base 18 (cf. Figure 5). The result of the tapering of the thread segments in the longitudinal direction is that the front face 10 has a clearance angle at each of its outer cutting edges (only the clearance angle α₁ on one side is identified).

The decreasing width of the thread segment along its length has the further effect that the cutting edges of the thread segment project on both sides over the extended envelope of the leading thread segment end and thus create directly acting cutting edges which are effective even if no bone material is forced back elastically into the thread groove 9 located in front of the cutting edges.

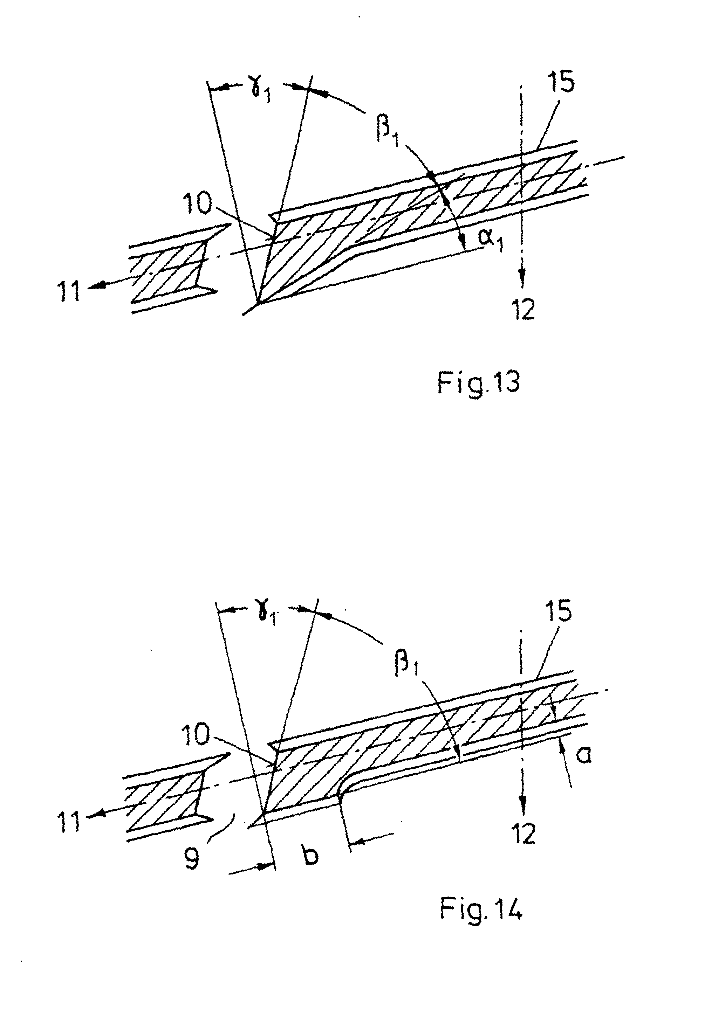

Figure 13 shows a view as in Figure 12 of an embodiment according to the invention. In the embodiment according to Figure 13, a clearance angle α₁, in combination with a directly acting wedge angle β₁ and a cutting rake γ₁, is provided only on the pole side, or that of the direction of screwing in, of the front face 10 of the thread segment.

According to an alternative aspect, the thread segment can also be realized, in the sectional representation of Figure 13, in such a way that it does not have a clearance angle α₁, but the cutting edge located at the front in the direction of screwing in is replaced by a cutting lip having the width b, as depicted in Figure 14, which in practice achieves an effect similar to a clearance angle. The thread segment is thus fashioned so that it is widened in the region of the cutting lip and is then recessed by a step a. The width b of the cutting lip can be e.g. 0.3 mm and the depth a behind the cutting lip e.g. 0.05 mm.

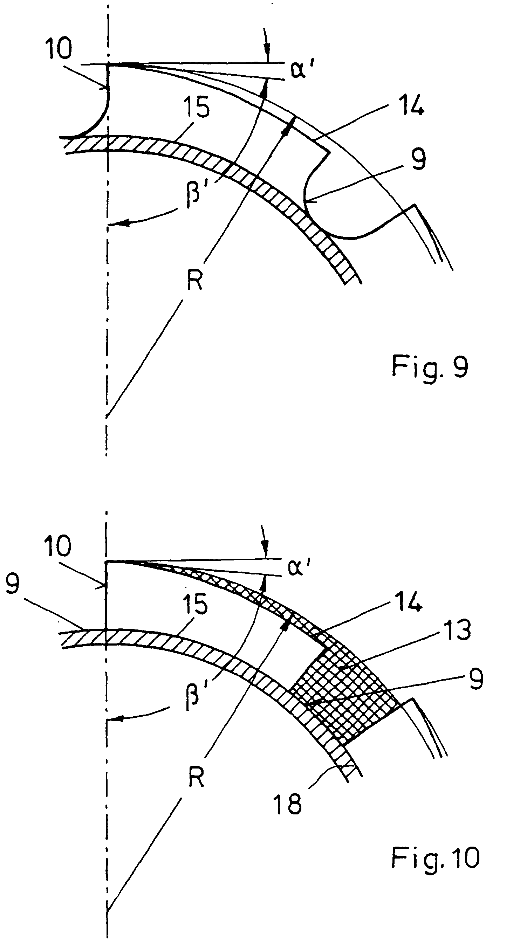

The figures 9 to 11 depict views of the thread according to a sectional representation through the plane A-B from Figure 5. In the case of the embodiment depicted in Figure 9, by contrast with the known thread depicted in Figure 6, the height of the thread contour 14 above the base 18 decreases. By this means, a clearance angle α' of the cutting edge of the thread segment is achieved in this sectional plane. The height of the thread segment above the thread root decreases in the course of the length of a thread segment, from the starting point to the end point in the direction of rotation.

As a result, however, the curved thread contour 14 with a continuously decreasing thread height acquires a greater thread segment surface by contrast with a straight, flattened thread contour as in Figure 3 and Figure 4, i.e., the loss surface 13 indicated in Figures 3 and 10 is minimized. The loss surface 13 is the surface which is effectively lost to the thread for bedding or anchoring in the bone. The effectuation of the clearance angle α' by a continuously curved thread contour with a decreasing height above the thread base is therefore advantageous in respect of a better anchorage of the screw socket in the bone.

Figures 10 and 11 depict alternative designs of the thread grooves 9, which result in different forms of the cutting edge. In the case of the embodiment depicted in Figure 11, the thread groove 9 is sunk obliquely so that, in this sectional view, the cutting edge has a cutting rake y'.

With its clearance angle α', this thread contour primarily compensates the geometrically determined increase in diameter after a thread crest which, in the absence of a clearance angle, i.e., α' = 0, would result in a clearance stoppage of the outer contour of the thread on the osseous socket.

## Claims

1. Screw socket for an artificial hip joint, with a self-cutting V-shaped, buttress, trapezoidal or round thread on a thread base of a screw socket body having a pole, wherein each thread segment, in the front region in the direction of rotation, is formed with a cutting edge with a clearance face so that, in the section in a plane which is located in the front region with parallel displacement relative to a plane tangential to the thread base (18), the said cutting edge has a clearance angle α₁ **characterized in that** the clearance angle α₁ is, in combination with a directly acting wedge angle β₁ and a cutting rake γ₁, provided only on the pole side of the face (10) of each thread segment.

2. Screw socket according to Claim 1, **characterized in that** the clearance angle α is greater than 0.01 (radian measure).

3. Screw socket according to one of Claims 1 to 2, **characterized in that** the cutting rake γ is greater than 0.01 (radian measure).

4. Screw socket according to one of the preceding Claims, **characterized in that** the front region, in the direction of rotation, of each thread segment is formed with a further cutting edge with a clearance face so that, in the profile longitudinal section (i.e., through a plane located, tangentially relative to the spatial spiral line of the thread course, in the front region of the thread segment), the thread segment has a clearance angle α'.

5. Screw socket according to Claim 4, **characterized in that** the further cutting edge is additionally formed with a cutting face so that, in the profile longitudinal section, the thread segment has a cutting rake γ'.

6. Screw socket according to Claim 4, **characterized in that** the clearance angle α' is effected through a continuously curved thread contour (14) whose height above the thread base (18) decreases.

7. Screw socket according to Claim 5, **characterized in that** the cutting rake γ' is greater than 0.01 (radian measure).

8. Screw socket for an artificial hip joint, with a self-cutting thread on a thread base of a screw socket body having a pole, **characterized in that** each thread segment, in the front region in the direction of rotation (11), is formed with a cutting edge with a clearance face so that, in the section in a plane which is located in the front region with parallel displacement relative to a plane tangential to the thread base (18), the said cutting edge has a cutting rake γ and, in this section, is widened in the direction to the pole only in the front region so that, due to the widening, the front cutting edge projects over the envelope of the leading thread segment in the direction of rotation.

## Patentansprüche

1. Schraubpfanne für ein künstliches Hüftgelenk, mit einem selbstschneidenden Spitz-, Sägezahn-, Trapez- oder Rundgewinde auf einer Gewindegrundfläche eines Schraubpfannengrundkörper mit einem Pol, wobei jedes Gewindesegment im in Drehrichtung vorderen Bereich mit einer Schneide mit einer Freifläche so geformt ist, dass sie im Schnitt in einer Ebene, die parallelversetzt zu einer Tangentialebene an die Gewindegrundfläche (18) im vorderen Bereich liegt, einen Freiwinkel α hat, **dadurch gekennzeichnet, dass** der Freiwinkel α₁ in Kombination mit einem direkt wirksamen Keilwinkel β₁ und einem Spanwinkel γ₁ nur auf der Polseite der Stirnfläche (10) jedes Gewindesegments vorgesehen ist.

2. Schraubpfanne nach Anspruch 1, **dadurch gekennzeichnet, dass** der Freiwinkel α größer als 0,01 (Bogenmaß) ist.

3. Schraubpfanne nach einem der Ansprüche 1 bis 2, **dadurch gekennzeichnet, dass** der Spanwinkel γ größer als 0,01 (Bogenmaß) ist.

4. Schraubpfanne nach einem der vorhergehenden Ansprüche, **dadurch gekennzeichnet, dass** der in Drehrichtung vordere Bereich des Gewindesegments mit einer weiteren Schneide mit einer Freifläche so geformt ist, dass das Gewindesegment im Profillängsschnitt (d.h. durch eine Ebene im vorderen Bereich des Gewindesegments tangential zur räumlichen Spirallinie des Gewindelaufs) einen Freiwinkel α' hat.

5. Schraubpfanne nach Anspruch 4, **dadurch gekennzeichnet, dass** die weitere Schneide ferner mit einer Spanfläche so geformt ist, dass das Gewindesegment im Profillängsschnitt einen Spanwinkel γ' hat.

6. Schraubpfanne nach Anspruch 4, **dadurch gekennzeichnet, dass** der Freiwinkel α' durch eine kontinuierlich gekrümmte Gewindekontur (14), deren Höhe über der Gewindegrundfläche (18) abnimmt, realisiert ist.

7. Schraubpfanne nach Anspruch 5, **dadurch gekennzeichnet, dass** der Spanwinkel γ' größer als 0,01 (Bogenmaß) ist.

8. Schraubpfanne für ein künstliches Hüftgelenk, mit einem selbstschneidenden Gewinde auf einer Gewindegrundfläche eines Schraubpfannengrundkörpers mit einem Pol, **dadurch gekennzeichnet, dass** jedes Gewindesegment im in Drehrichtung (11) vorderen Bereich mit einer Schneide mit einer Freifläche so geformt ist, dass sie im Schnitt in einer Ebene, die parallel versetzt zu einer Tangentialebene an die Gewindegrundfläche (18) im vorderen Bereich liegt, einen Spanwinkel γ hat, und in diesem Schnitt im vorderen Bereich nur in Richtung des Pols verbreitert ist, so dass die vordere Schneidkante auf Grund der Verbreiterung über die Einhüllende des in Drehrichtung vorauslaufenden Gewindesegmentendes hinausragt.

## Revendications

1. Cupule à vis pour une articulation de hanche artificielle, avec un épaulement autotaraudeur en forme de V, un filetage trapézoïdal ou rond sur une base de filetage d'un corps de cupule à vis ayant une barre, dans laquelle chaque segment de filetage, dans la région avant dans le sens de rotation, est formé avec une arête coupante avec une face de dégagement de telle sorte que, dans la section dans un plan qui se situe dans la région avant avec un déplacement parallèle par rapport à un plan tangentiel à la base du filetage (18), ladite arête coupante présente un angle de dégagement α₁, **caractérisée en ce que** l'angle de dégagement α₁, en combinaison avec un angle de prise à action directe β₁ et un angle d'inclinaison de coupe γ₁, n'est fourni que sur le côté de la barre de la face (10) de chaque segment de filetage.

2. Cupule à vis selon la revendication 1, **caractérisée en ce que** l'angle de dégagement α est supérieur à 0,01 (mesure radian).

3. Cupule à vis selon l'une quelconque des revendications 1 à 2, **caractérisée en ce que** l'angle d'inclinaison de coupe γ est supérieur à 0,01 (mesure radian).

4. Cupule à vis selon l'une quelconque des revendications précédentes, **caractérisée en ce que** la région avant, dans le sens de rotation, de chaque segment de filetage est formée avec une autre arête coupante avec une face de dégagement de telle sorte que, dans la section longitudinale du profil (c'est-à-dire, à travers un plan situé, de manière tangentielle par rapport à la ligne spirale spatiale de la direction du filetage, dans la région avant du segment de filetage), le segment de filetage a un angle de dégagement α'.

5. Cupule à vis selon la revendication 4, **caractérisée en ce que** l'autre arête coupante est en outre formée avec une face coupante de telle sorte que, dans la section longitudinale du profil, le segment de filetage a un angle d'inclinaison de coupe γ'.

6. Cupule à vis selon la revendication 4, **caractérisée en ce que** l'angle de dégagement α' est réalisé à travers un contour de filetage (14) incurvé de manière continue dont la hauteur au-dessus de la base du filetage (18) diminue.

7. Cupule à vis selon la revendication 5, **caractérisée en ce que** l'angle d'inclinaison de coupe γ' est supérieur à 0,01 (mesure radian).

8. Cupule à vis pour une articulation de hanche artificielle, avec un filetage autotaraudeur sur une base de filetage d'un corps de cupule à vis ayant une barre, **caractérisée en ce que** chaque segment de filetage, dans la région avant dans le sens de rotation (11), est formé avec une arête coupante avec une face de dégagement de telle sorte que, dans la section dans un plan qui se situe dans la région avant avec un déplacement parallèle par rapport à un plan tangentiel à la base du filetage (18), ladite arête coupante a un angle d'inclinaison de coupe γ et, dans cette section, est élargie dans la direction de la barre uniquement dans la région avant, de telle sorte que, en raison de l'élargissement, l'arête coupante avant surplombe l'enveloppe du segment de filetage avant dans le sens de rotation.
